# EUROPEAN PATENT SPECIFICATION

(11) **EP 4 036 571 B1**
(45) Date of publication and mention of the grant of the patent: **11.12.2024**
(21) Application number: 21211931.7
(22) Date of filing: 02.12.2021
(51) Int. Cl.: G01N 33/02, G01N 21/64, G01N 31/22, G01N 21/77

(54) **FRESHNESS LABEL STRUCTURE AND FRESHNESS LABEL**
FRISCHEETIKETTSTRUKTUR UND FRISCHEETIKETT
STRUCTURE D'ÉTIQUETTE FRAÎCHEUR ET ÉTIQUETTE FRAÎCHEUR

(30) Priority: 01.02.2021 JP 2021014448
(43) Date of publication of application: 03.08.2022
(73) Proprietor: Toshiba TEC Kabushiki Kaisha, Tokyo 141-8562 (JP)
(72) Inventor: Akiyama, Ryozo, Shinagawa-ku, Tokyo 141-8562 (JP); Fukazawa, Taishi, Shinagawa-ku, Tokyo 141-8562 (JP); Ishikawa, Daisuke, Shinagawa-ku, Tokyo 141-8562 (JP)
(74) Representative: Bandpay & Greuter

(56) References cited:
- WO-A1-2020/021053
- JP-A- 2017 058 233
- JP-B1- 6 026 628
- US-A1- 2006 240 565
- US-A1- 2017 307 579

## Description

### FIELD

Embodiments described herein relate generally to a freshness label structure with a sealing member and a freshness label with a sealing member.

### BACKGROUND

Fresh foods such as marine products, fruit and vegetable products, livestock products, or the like have poor storage properties at a room temperature and are likely to deteriorate, such that a method for evaluating freshness thereof is required. As a method for evaluating the freshness of fresh food, various methods such as a sensory evaluation method, a chemical evaluation method, a physical evaluation method, a microbial method, or the like are tried. The sensory evaluation method is a method for evaluating appearance, odor, tactile sensation such as tension or the like of the fresh food. The chemical evaluation method is performed by gas chromatography mass spectrometry of a chemical component generated by deterioration and putrefaction of the fresh food, and liquid chromatography mass spectrometry of nucleic acid-related compound. The physical evaluation method is a method for performing evaluation based on physical indexes such as a rigidity index (a RI value), breaking strength, texture, impedance, or the like of the fresh food. The microbial method is a method for investigating the number of general viable bacteria, the number of putrefactive bacteria, the number of pathogenic bacteria, or the like contained in the fresh food.

While the sensory evaluation method does not require an expensive measuring device and can evaluate freshness in a short time, there is a problem that a result of the freshness varies depending on an evaluator. While the chemical evaluation method, the physical evaluation method, and the microbial method are not likely to cause variations in the result thereof by the evaluator, it is not easy to perform the evaluation due to the requirement of equipment and facilities, and measurement may take time. That is, such methods are required to improve simplicity or accuracy.

In order to solve the above-described problems, a freshness label using an aggregation-induced phosphor is proposed. The freshness label can evaluate the freshness of food by measuring fluorescence intensity of the label. Here, the aggregation-induced phosphor is a compound indicating aggregation-induced emission (AIE). The aggregation-induced phosphor indicates weak or no fluorescence in a state where each molecule is dissolved in a solution such as a soluble organic solvent or the like. For example, molecules of the aggregate-induced phosphor in a dissolved state aggregate to form an aggregate when reacting with a chemical component released by putrefaction and deterioration of the fresh food, and the aggregate emits strong fluorescence. The aggregation-induced phosphor is desirably designed to be based on a structure including a functional group that reacts with a putrefactive component. For example, if an amine component, which is one of the putrefactive components, reacts with the aggregation-induced phosphor, it is desirable to have a carboxylic acid group.

### DESCRIPTION OF THE DRAWINGS

FIG. 1 is a perspective view schematically illustrating a freshness label structure provided in a freshness label structure with a sealing member according to an embodiment;
FIG. 2 is an enlarged cross-sectional view of the freshness label structure;
FIG. 3 is a top view schematically illustrating an example of a freshness label with a sealing member;
FIG. 4 is a cross-sectional view of the freshness label with the sealing member;
FIG. 5 is a cross-sectional view schematically illustrating another example of the freshness label with the sealing member;
FIG. 6 is a cross-sectional view schematically illustrating another example of the freshness label with the sealing member;
FIG. 7 is a diagram illustrating a quenching mechanism of an aggregation-induced phosphor;
FIG. 8 is a perspective view illustrating an example of a packaged food including the freshness label;
FIG. 9 is a diagram illustrating a decomposition process of a nucleic acid-related substance;
FIG. 10 is a diagram illustrating a method for calculating a K value;
FIG. 11 is a top view schematically illustrating another example of the freshness label with the sealing member;
FIG. 12 is a diagram illustrating images obtained in an embodiment using a horse mackerel; and
FIG. 13 is a diagram illustrating images obtained in an embodiment related to presence or absence of a moisturizer.

### DETAILED DESCRIPTION

The invention is set out in the appended set of claims.

A problem to be solved by embodiments is to provide a freshness label with a sealing member capable of simply confirming freshness of food with high accuracy, and a freshness label structure with a sealing member for implementing the freshness label with the sealing member.

In general, according to one embodiment, a freshness label structure with a sealing member is provided. A freshness label structure provided in the freshness label structure with the sealing member includes a base material and a phosphor layer including an aggregation-induced phosphor fixed to the base material. A sealing member provided in the freshness label structure with the sealing member is impermeable to aqueous liquid, seals the freshness label structure, and includes a portion formed of a porous member impermeable to aqueous liquid and permeable to gas.

According to another embodiment, a freshness label with a sealing member is provided. The freshness label with the sealing member (10, 20, 30) includes the freshness label structure with the sealing member (100) according to the embodiment, and water carried on the freshness label structure (1).

According to careful research by inventors, it is found out that there is room for improvement in accuracy of a freshness label including an aggregation-induced phosphor. That is, the freshness label including the aggregation-induced phosphor dissolved in an organic solvent is provided, for example, in a container in which fresh food is sealed and in the vicinity of the fresh food. The fresh food is food containing a high water content. Therefore, it is considered that a water vapor pressure in the fresh food and the container in which the freshness label is sealed reaches a saturated water vapor pressure over time. Since the aggregation-induced phosphor is a water-insoluble substance, it is considered that the aggregation-induced phosphor is not easily affected by water. However, the inventors find out that fluorescence of the freshness label decreases as the water vapor pressure in the container increases. That is, since a fluorescence intensity of the freshness label is affected by water in a gas phase, under an environment where humidity gradually increases, an influence caused by a chemical component released by putrefaction and deterioration of the fresh food is not accurately reflected in the fluorescence intensity.

If the freshness label is sealed in a container with fresh food such as fresh fish, raw meat, or the like, the freshness label is often directly covered with a drip which is an aqueous liquid component released from the fresh food. The freshness label may be used to manage the freshness of landed fresh fish stored in a cold storage box, and ice water, which is contained as a cold insulation material, scatters during transportation of the cold storage box, such that the freshness label is directly covered with a water droplet. The water droplet on the freshness label directly affects a fluorescence effect of the freshness label. In the freshness label that evaluates the freshness of food by measuring the fluorescence intensity of the freshness label, particularly, it is important to provide a shielding property against an aqueous liquid in order to accurately measure the freshness. Embodiments are performed based on the above-described findings.

Hereinafter, embodiments will be described in detail with reference to the drawings. Components that perform the same or similar functions will be denoted by the same reference signs throughout all drawings, and duplicate description will be omitted.

A freshness label structure with a sealing member 100 according to an embodiment includes a freshness label structure and an aqueous liquid impermeable sealing member that seals the freshness label structure. In the following, the term "freshness label structure" simply indicates a freshness label structure that does not include a sealing member, and is distinguished from the freshness label structure with the sealing member including the sealing member.

The freshness label structure includes a base material and a phosphor layer. The phosphor layer contains an aggregation-induced phosphor fixed to the base material. Since the freshness label structure contains a solid aggregate-induced phosphor, the freshness label structure indicates strong fluorescence.

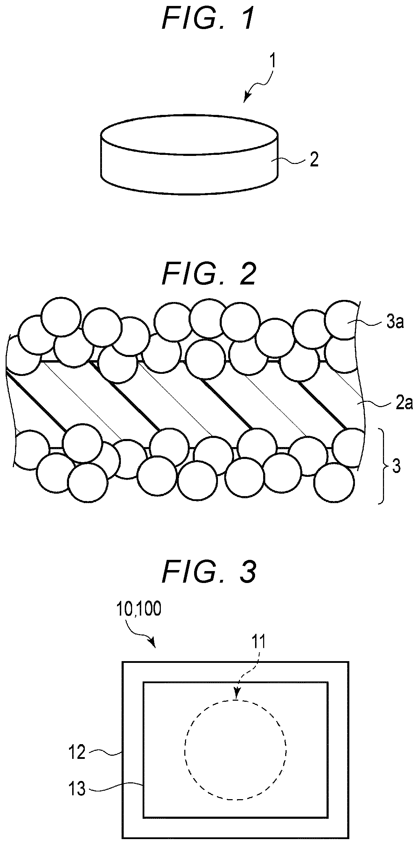

FIG. 1 is a perspective view schematically illustrating a freshness label structure provided in a freshness label structure with a sealing member 100 according to an embodiment. A freshness label structure 1 illustrated in FIG. 1 includes a base material 2 and a phosphor layer which is not illustrated. FIG. 2 is an enlarged cross-sectional view of the freshness label structure illustrated in FIG. 1. The freshness label structure 1 illustrated in FIGS. 1 and 2 is an example in which filter paper is used as the base material 2. A phosphor layer 3 is carried on a fiber 2a of the base material 2. The phosphor layer 3 contains an aggregation-induced phosphor 3a fixed to the fiber 2a of the base material 2.

Shape, material, or the like of the base material 2 are not limited as long as the base material 2 can be impregnated with water. The base material 2 has, for example, a porous body or a network structure. The shape of the base material 2 may be a circular shape as illustrated in FIG. 1 or a polygonal shape. A thickness of the base material 2 is, for example, 0.1 mm or more and 1.0 mm or less. The thickness thereof is not particularly limited as long as an amount of fluorescence emitted from the aggregation-induced phosphor can be secured, or the thickness thereof may be formed to the extent that the thickness does not interfere with a reaction between the aggregation-induced phosphor and a putrefactive component inside the base material.

The base material 2 is a fibrous material that contains, for example, synthetic fiber, inorganic fiber, natural fiber, or a mixture thereof. Examples of the synthetic fiber include polyolefin fiber and cellulosic fiber. Examples of the inorganic fiber include glass fiber, metal fiber, alumina fiber, and activated carbon fiber. Examples of the natural fiber include wood pulp and hemp pulp. The base material 2 is desirably a layer formed of glass fiber.

The phosphor layer 3 contains the aggregation-induced phosphor 3a, and is desirably formed of only the aggregation-induced phosphor 3a. The phosphor layer 3 is carried on the base material 2. The phosphor layer 3 is desirably carried on a thin layer on a surface of the fiber 2a of the base material 2.

It is desirable that a thickness of the phosphor layer 3 is set to a thickness in which a fluorescence intensity thereof becomes sufficiently small if the phosphor layer 3 is left in an environment at 25°C and 100% relative humidity. Here, regarding the fact that the fluorescence intensity becomes sufficiently small, for example, if the fluorescence intensity if the phosphor layer 3 is left in the environment at 25°C and 100% relative humidity is calculated as a relative value in which the fluorescence intensity if the phosphor layer 3 is left in an environment at 10°C and 20% relative humidity is set to 100%, the fluorescence intensity becomes 30% or less. The fluorescence intensity is measured, for example, by using a photodetector such as a photodiode or the like, or by using imaging elements such as a complementary metal-oxide-semiconductor (CMOS) image sensor, a charge-coupled device (CCD) image sensor, or the like. If a color type imaging element is used, for example, an arithmetic average of a gradation value of RBG is regarded as the fluorescence intensity.

The thickness of the phosphor layer 3 can affect a fluorescence intensity of the freshness label. That is, if the phosphor layer 3 is appropriately thickened, the fluorescence intensity of the freshness label tends to increase. On the other hand, if the phosphor layer 3 is formed to be excessively thick, a change in fluorescence intensity depending on a change in freshness becomes small. The thickness of the phosphor layer 3 is desirably 30 nm or less, and is more desirably 20 nm or less. It is desirable that the thickness of the phosphor layer 3 is adjusted within a range in which the change in freshness can be easily confirmed, depending on an amount of a chemical component (hereinafter referred to as a target component) released due to putrefaction and deterioration of fresh food. The thickness of the phosphor layer 3 can be confirmed by, for example, a transmission electron microscopy (TEM).

Here, the "target component" will be described. Fresh food can release one or more target components into a gas phase if the fresh food putrefies and deteriorates. The target component includes, for example, acidic components such as aldehyde, carboxylic acid, or the like, alcohol, basic components such as ammonia, amine, or the like, ester, and ketone. Aldehyde includes, for example, hexanal, 3-methylbutanal, nonanal, isovaleraldehyde, or a mixture thereof. The carboxylic acid includes, for example, formic acid, acetic acid, isovaleric acid, or a mixture thereof. Amine includes, for example, trimethylamine, dimethylamine, 1,2-ethylenediamine, 1,3-propanediamine, 1,4-butanediamine, 1,5-pentanediamine, 1,6-hexanediamine, spermidine, spermine, histamine, tryptamine, or a mixture thereof. Alcohol includes, for example, ethanol, isopropyl alcohol, 3-methyl-1-butanol, 1-pentanol, 1-butanol, or a mixture thereof. Ester includes, for example, ethyl acetate, methyl acetate, ethyl propionate or a mixture thereof. Ketone includes, for example, methyl ethyl ketone, acetone, mercaptoacetone, or a mixture thereof.

The aggregation-induced phosphor 3a may form the phosphor layer 3 as a granular layer as illustrated in FIG. 2, and may form the phosphor layer 3 as a continuous film without a gap. In the phosphor layer 3 as the granular layer, each particle contains a plurality of molecules of the aggregation-induced phosphor 3a, and the number of molecules of the aggregation-induced phosphor 3a located on the shortest straight line connecting each location in the particle and a particle surface is, for example, 10 or less.

The aggregation-induced phosphor 3a desirably contains a phosphor including a polar functional group. The aggregation-induced phosphor 3a including the polar functional group easily reacts with the target component, such that the accuracy of freshness evaluation using the freshness label can be improved. The aggregation-induced phosphor 3a tends to be highly soluble or dispersible in water. The polar functional group may be an acidic functional group or a basic functional group. Examples of the acidic functional group include a carboxyl group and a sulfo group. Examples of the basic functional group include a hydroxyl group and an amino group. The aggregation-induced phosphor 3a may contain a plurality of types of acidic functional groups or basic functional groups. The aggregation-induced phosphor 3 a desirably contains two or more carboxyl groups in one molecule.

As the aggregation-induced phosphor 3a, the one including a tetra-phenylethylene skeleton represented in structural formula (2), a silole skeleton represented in structural formula (3), or a phosphole oxide skeleton represented in structural formula (4) can be used. Each of these compounds may be cis, trans, or a mixture of the cis and the trans.

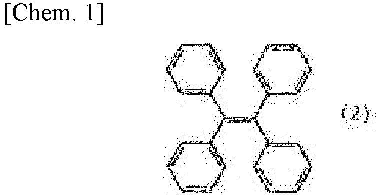

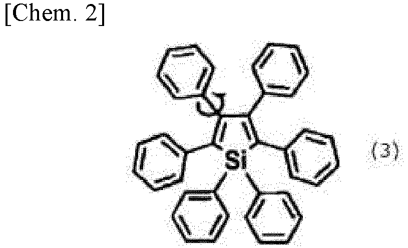

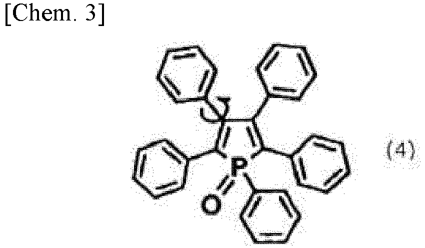

From a viewpoint that the aggregation-induced phosphor 3a is particularly excellent in reactivity with the target component, the aggregation-induced phosphor 3a desirably contains a tetra-phenyl ethylene derivative represented by the following general formula (I).

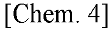

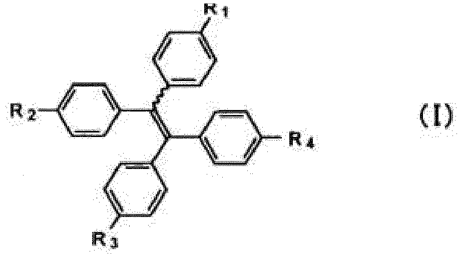

In general formula (I), R₁, R₂, R₃, and R₄ are independent of each other, and selected from a group formed of -LiMi, -(CH₂)ₘ-L₂M₂, -X-(CH₂)ₙ-L₃M₃, -Y-(CH₂)ₒ-Z-(CH₂)ₚ-L₄M₄ (here, L₁, L₂, L₃, and L₄ are independent of each other and represent -CO₂- or -SO₃-, M₁, M₂, M₃, and M₄ are independent of each other and represent a hydrogen atom or a cation, X, Y, and Z are independent of each other and represent -O-, -NH-, or -S-, and m, n, o, and p are independent of each other and represent integers 1 to 6), a hydrogen atom, a halogen atom, a hydroxyl group, a nitro group, a carbamoyl group, an alkyl groups having a carbon number of 1 to 6, a haloalkyl group having a carbon number of 1 to 6, an alkenyl group having a carbon number of 2 to 6, a cycloalkyl group having a carbon number of 3 to 10, an alkyloxy group having a carbon number of 1 to 6, an acyl group having a carbon number of 2 to 6, an amino group, an alkylamino group having a carbon number of 1 to 6, an aryl group having a carbon number of 6 to 10, and a heteroaryl group having a carbon number of 5 to 10, and at least two of R₁, R₂, R₃ and R₄ are independent of each other, and are selected from a group formed of -L₁M₁, -(CH₂)ₘ-L₂M₂, -X-(CH₂)ₙ-L₃M₃, and -Y-(CH₂)ₒ-z-(CH₂)ₚ-L₄M₄ (Here, L₁, L₂, L₃, L₄, M₁, M₂, M₃, M₄, X, Y, Z, m, n, o, and p are as described above).

Specific examples of the tetraphenylethylene derivative include compounds represented by the following structural formulas (5), (6), (7) and (8).

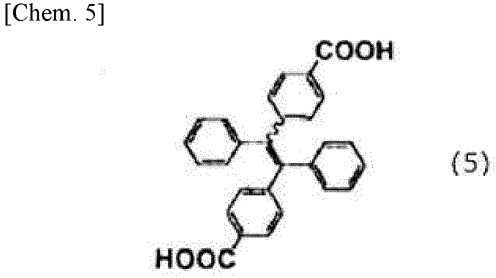

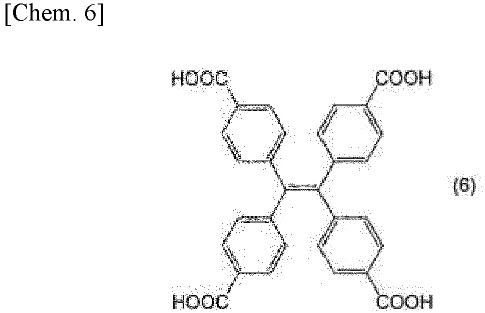

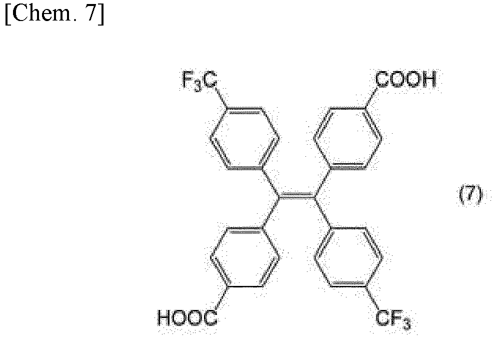

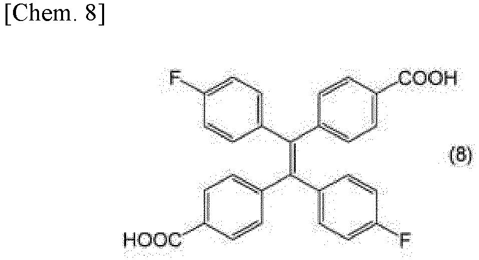

For example, the freshness label structure 1 is manufactured by the following method. First, the aggregation-induced phosphor 3a is dissolved in an organic solvent to prepare a treatment liquid. Any type of the organic solvent may be used as long as the organic solvent can dissolve the aggregation-induced phosphor 3a, and the organic solvent having a low evaporation temperature is desirable. As the organic solvent, for example, ethanol is used. For example, concentration of the aggregation-induced phosphor 3a in the treatment liquid is 50 µM (molar mass) or more and 1 mM (molar mass) or less if the compound represented by the structural formula (6) is used.

Next, after the base material 2 is immersed in the treatment liquid to be impregnated with the treatment liquid, the base material 2 is pulled up from the treatment liquid and dried. Instead of immersing the base material 2 in the treatment liquid, the base material 2 may be impregnated with the treatment liquid by dropping the treatment liquid by using a dropper or the like. As such, the freshness label structure 1 is obtained. The freshness label structure 1 typically does not contain the organic solvent.

The freshness label structure 1 may further contain a moisturizer. If the freshness label structure 1 contains the moisturizer, in the freshness label formed by carrying water on the freshness label structure 1, evaporation of water can be prevented. As described above, since the freshness label with the sealing member using the freshness label structure 1 containing the moisturizer has excellent water retention property of water contained in the freshness label, an initial fluorescence state can be maintained until the freshness label with the sealing member is used for evaluating the freshness of food.

Examples of the moisturizer include glycerin, propylene glycol, triacetin, sorbitol, or the like. The freshness label structure containing the moisturizer can be obtained by adding a predetermined amount of the moisturizer in the preparation of the treatment liquid for dissolving the aggregation-induced phosphor 3a in the organic solvent, with respect to the above-described method for manufacturing the freshness label structure 1. The concentration of the moisturizer in the treatment liquid may be, for example, 1% by mass or more and 10% by mass or less if glycerin is used as the moisturizer.

The freshness label includes the freshness label structure 1 illustrated in FIG. 1 and water held by the freshness label structure 1. The freshness label with the sealing member is obtained by carrying water on the freshness label structure 1 provided in the freshness label structure with the sealing member 100. In the following, the term "freshness label" simply indicates a freshness label that does not include a sealing member, and is distinguished from a freshness label with a sealing member including the sealing member. The freshness label with the sealing member and the freshness label structure with the sealing member commonly include the sealing member. The freshness label with the sealing member will be described below, and the description thereof can be substituted for the description of the freshness label structure with the sealing member by replacing the "freshness label" with the "freshness label structure".

The sealing member provided in the freshness label with the sealing member according to the embodiment is impermeable to aqueous liquid, and includes a portion formed of a porous member which is impermeable to aqueous liquid and permeable to gas. The sealing member has a function of preventing aqueous liquid from directly contacting the phosphor layer (hereinafter, also referred to as "waterproof function") by sealing the freshness label from an external atmosphere surrounding the freshness label (hereinafter referred to as "surrounding atmosphere"). In addition to the waterproof function, the porous member provided in at least a part of the sealing member has a function of allowing the chemical component (the target component) released due to the putrefaction and deterioration of food such as fresh food or the like to contact the phosphor layer via the porous member by gas permeability.

Here, the "aqueous liquid" is water as a liquid or an aqueous solution containing water, and examples thereof include water, ice water, seawater, a drip released from fresh food, or the like.

A sealing structure of the freshness label by the sealing member is not particularly limited as long as the freshness label can be sealed from the surrounding atmosphere, the portion formed of the porous member, which is impermeable to aqueous liquid and permeable to gas, provided in the sealing member can contact the surrounding atmosphere, and the target component can contact the freshness label via the porous member.

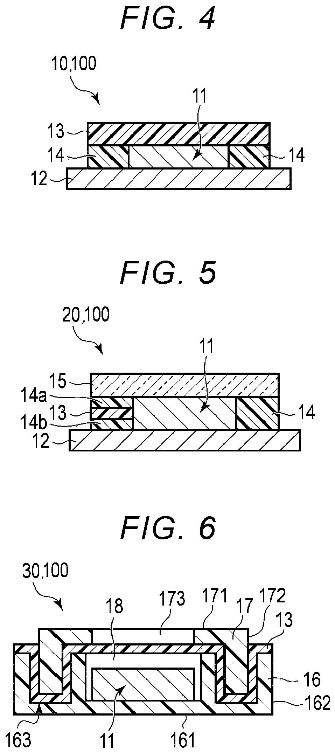

Hereinafter, the freshness label with the sealing member according to the embodiment will be described with reference to the drawings. FIG. 3 is a top view schematically illustrating an example of the freshness label with the sealing member according to the embodiment, and FIG. 4 is a cross-sectional view thereof. A freshness label with a sealing member 10 illustrated in FIGS. 3 and 4 includes a freshness label 11 and a sealing member formed of a retaining body 12, a porous member 13, and a joining member 14 in a form of sealing the freshness label 11 from the surrounding atmosphere. The freshness label 11 includes the freshness label structure 1 illustrated in FIGS. 1 and 2 and water, which is not illustrated, carried on the freshness label structure 1. The retaining body 12, the porous member 13, and the joining member 14 forming the sealing member are impermeable to aqueous liquid, and among the retaining body 12, the porous member 13, and the joining member 14, the porous member 13 is also permeable to gas. FIG. 3 does not illustrate the joining member 14 illustrated in FIG. 4.

The retaining body 12 forms the sealing member that seals the freshness label 11 together with the porous member 13 and the joining member 14. The retaining body 12 supports the freshness label 11 in a form in which the porous member 13 can contact the freshness label 11. A material of the retaining body 12 may be impermeable to aqueous liquid. A material that does not emit fluorescence from the retaining body 12 itself is desirable, and a material that emits fluorescence is not particularly limited as long as the material does not affect fluorescence observation of the freshness label 11. Examples of the material of the retaining body 12 include glass, resin (for example, acrylic resin, PET, PP, PE, cycloolefin polymer, or the like), paper, or the like. Particularly, since the cycloolefin polymer hardly emits its own fluorescence, the cycloolefin polymer is desirable as a material for the retaining body 12 if the fluorescence of the freshness label is observed via the retaining body 12. If the fluorescence observation of the freshness label is performed via the porous member 13, and if the glass and resin are blackened not to transmit light, the porous member 13 is less affected by the fluorescence of the base material such that it is also possible to expand selectivity of the material.

The porous member 13 covers a main surface on an opposite side of a main surface supported by the retaining body 12 of the freshness label 11. The porous member 13 is in contact with the surrounding atmosphere, and the target component can contact the freshness label 11 via the porous member 13. In another example, the whole sealing member may be formed of the porous member 13 such that the whole freshness label 11 may be sealed by the porous member 13. A shape is not limited to a shape of the porous member 13 and is not limited to a layered shape as illustrated in FIGS. 3 and 4. As long as the functions of aqueous liquid impermeability and gas permeability can be performed, for example, the shape may have other shapes such as a cylindrical shape, a bag shape, or a shape to be integrated with a food packaging material such as a wrap film, or the like.

A material of the porous member 13 is not particularly limited and can use any material as long as the material thereof is a material having aqueous liquid impermeability and gas permeability. As such a material, a hydrophobic polytetrafluoroethylene (PTFE) membrane filter has low wettability to aqueous liquid, has good liquid impermeability by water repellency, and has good gas permeability with good porosity, such that the hydrophobic polytetrafluoroethylene (PTFE) membrane filter is particularly desirable as the material for the porous member 13. A pore size of the hydrophobic PTFE membrane filter is, for example, desirably 1 µm to 10 µm, and is more desirably 3 µm to 5 µm. Since the PTFE membrane filter does not show fluorescence from the PTFE membrane filter itself, the PTFE membrane filter does not interfere with the fluorescence observation of the fluorescent layer, and selects a filter having a thickness capable of transmitting excitation light and fluorescence to the extent necessary for the purpose of determining the freshness label. A cooking sheet in which a surface of the paper is processed with silicon and Teflon (registered trademark) can also be used as the porous member 13.

The joining member 14 seals the freshness label 11 in a form in which the freshness label 11 is sandwiched between the retaining body 12 and the porous member 13 by joining the retaining body 12 and the porous member 13. The joining member 14 may be a known adhesive such as a thermocompression bonding adhesive or the like, and an adhesive tape. An intensity of a waterproof structure to the freshness label 11 by the sealing member including the joining member 14 may be set according to a period of use or the like of the freshness label with the sealing member 10, and after the determination purpose by the freshness label with the sealing member 10 is achieved, adhesion of the joining member 14 may deteriorate.

Hereinafter, another example of the freshness label with the sealing member will be described. FIG. 5 is a cross-sectional view schematically illustrating another example of the freshness label with the sealing member according to the embodiment. A freshness label with a sealing member 20 illustrated in FIG. 5 includes a freshness label 11 and a sealing member formed of a retaining body 12, a porous member 13, joining members 14, 14A, and 14B, and film 15 in a form of sealing the freshness label 11 from the surrounding atmosphere.

A biggest difference between the freshness label with the sealing member 20 and the freshness label with the sealing member 10 illustrated in FIGS. 3 and 4 is that disposition of the porous member 13 is different. In the freshness label with the sealing member 20, the porous member 13 is disposed between the joining members 14a and 14b for joining the retaining body 12 and the film 15, and is joined to the retaining body 12 and the film 15. The film 15 is disposed at a location where the porous member 13 is disposed in the freshness label with the sealing member 10. The film 15 is selected from non-fluorescent and aqueous liquid impermeable films having a material and thickness capable of transmitting excitation light and fluorescence to the extent necessary for the purpose of determining the freshness label. The film 15 is desirably a non-fluorescent and aqueous liquid impermeable transparent film. Examples of the film 15 include a cycloolefin polymer or the like.

As another example, which is not illustrated, of the freshness label with the sealing member according to the embodiment, an example in which the disposition of the porous member 13 is changed in the freshness label with the sealing member 20 illustrated in FIG. 5 can be provided. For example, another example can provide a freshness label with a sealing member including a sealing structure in which an opening, in which the freshness label 11 contacts the surrounding atmosphere, is provided in a part of the retaining body 12 or the film 15, and the porous member 13 is disposed in the opening.

FIG. 6 is a cross-sectional view schematically illustrating another example of the freshness label with the sealing member according to the embodiment. A freshness label with a sealing member 30 illustrated in FIG. 6 includes a freshness label 11 and a sealing member formed of a first housing member 16, a second housing member 17, and a porous member 13 in a form of sealing the freshness label 11 from the surrounding atmosphere. The first housing member 16 and the second housing member 17 are impermeable to aqueous liquid.

The first housing member 16 is formed of a first bottom wall 161 and a first peripheral wall 162 that rises along a peripheral edge of the first bottom wall 161, and a groove 163 is provided at an upper end of the first peripheral wall 162. The second housing member 17 includes a second bottom wall 171 and a second peripheral wall 172 that rises along a peripheral edge of the second bottom wall 171, and the second bottom wall 171 includes an opening 173 in a central portion surrounded by the periphery.

The freshness label with the sealing member 30 is formed by fitting the second peripheral wall 171 of the second housing member 17 into the groove 163 provided in the first peripheral wall 162 of the first housing member 16 in a state where the porous member 13 is disposed between the first housing member 16 and the second housing member 17. The porous member 13 is in contact with the surrounding atmosphere at the opening 173 of the second housing member 171. The freshness label 11 is disposed in an internal space 18 formed by the first housing member 16 and the porous member 13, thereby preventing invasion of aqueous liquid from the outside. Meanwhile, the freshness label 11 can contact the target component via the porous member 13 which is in contact with the surrounding atmosphere. An intensity of a waterproof structure to the phosphor layer 3 by the sealing member including the first housing member 16 and the second housing member 17 may be set according to a period of use or the like of the freshness label with the sealing member 30, and after the determination purpose of the freshness label with the sealing member 30 is achieved, a housing intensity may deteriorate.

Next, a method for manufacturing the freshness label with the sealing member will be described with reference to the freshness label with the sealing member 10 illustrated in FIGS. 3 and 4 described above as an example.

First, a step of manufacturing the freshness label 11 provided in the freshness label with the sealing member 10 will be described. The freshness label 11 is formed by carrying water on the freshness label structure 1, and in the manufacturing thereof, the freshness label structure 1 is immersed in water to be impregnated with water, and then pulled out of the water. The freshness label structure 1 may be impregnated with water by dropping water thereon by using a dropper or the like, or the freshness label structure 1 may contain water by exposing the freshness label structure 1 to water vapor. As a type of water, distilled water, pure water, ion-exchanged water, or a mixture thereof can be used.

The freshness label structure 1 (the freshness label 11) containing water is pulled out of water and placed on the retaining body 12. Next, the joining member 14 is disposed on the retaining body 12 to surround the freshness label 11, and the porous member 13 is covered thereon to form a waterproof structure by a sealing material. If the joining member 14 is a thermocompression bonding film, by applying an appropriate pressure on a heated hot plate, the retaining body 12 and the porous member 13 can desirably adhere to each other.

A step of carrying water on the freshness label structure 1 and manufacturing the freshness label 11 may be performed after the freshness label structure 1 is sealed by the sealing member. In other words, the freshness label structure with the sealing member 100 including the freshness label structure 1 is manufactured, and next, the freshness label with the sealing member 10 may be obtained by carrying water on the freshness label structure 1. As a method for carrying water on the freshness label structure 1 after sealing the freshness label structure 1 by the sealing member, the freshness label structure with the sealing member 100 may be exposed to water vapor, or the freshness label structure with the sealing member 100 may be immersed in water as liquid. In the latter case, the process can be performed in a short time.

As described above, the freshness label 11 provided in the freshness label with the sealing member according to the embodiment includes the freshness label structure 1 and water carried on the base material 2. A content of water may be an amount that causes the fluorescence of the freshness label 11 to become weak or non-fluorescent. That is, the freshness label structure 1 contains water, such that the fluorescence becomes weak or non-fluorescent. The present inventors consider that the mechanism is described as follows.

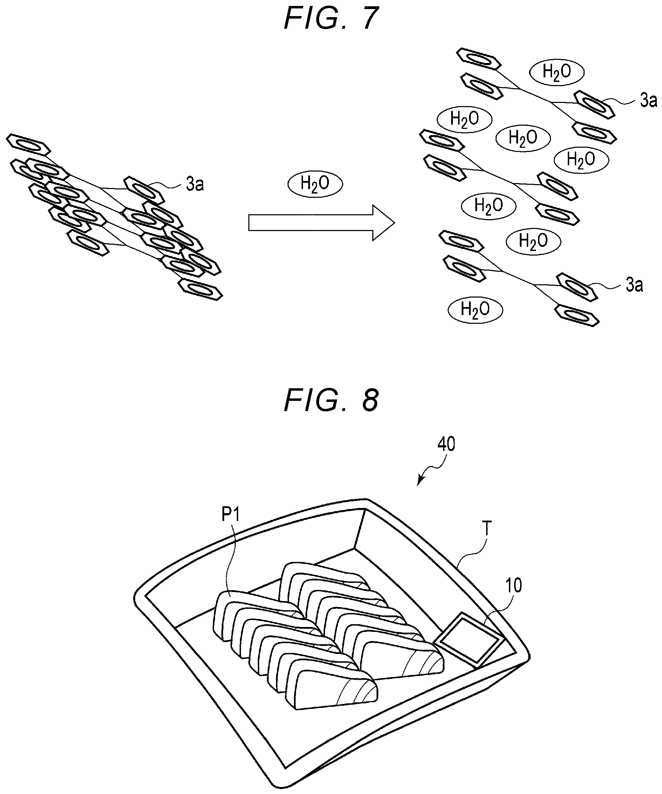

FIG. 7 is a diagram illustrating an example of a quenching mechanism of the aggregation-induced phosphor. As illustrated in FIG. 7, if an aggregate of molecules of the aggregation-induced phosphor 3a contacts water, water molecules enter a gap between the molecules of the aggregation-induced phosphor 3a, and a distance between the molecules becomes large. Alternatively, if water contacts the aggregate of the molecules of the aggregation-induced phosphor 3a, conformation of the molecules of the aggregation-induced phosphor 3a changes. If the arrangement of the molecules of the aggregation-induced phosphor 3a is changed as such, the fluorescence of the aggregation-induced phosphor 3a is weakened.

In the freshness label structure 1 illustrated in FIG. 2, water almost does not exist, such that the molecules of the aggregation-induced phosphor 3a form a layered structure. The aggregation-induced phosphor 3a in which the molecules are disposed as such can emit fluorescence at a relatively high intensity. On the other hand, in the freshness label 11, since the freshness label 11 is impregnated with water, water molecules enter the gap between the molecules of the aggregation-induced phosphor 3a. Therefore, the freshness label 11 emits fluorescence with a weaker intensity than that of the freshness label structure 1, or does not emit the fluorescence.

Particularly, the aggregation-induced phosphor 3a including a polar functional group such as a carboxyl group or the like has high affinity with water. Therefore, it is considered that the aggregation-induced phosphor 3a including the polar functional group is likely to have weak or no fluorescence if water is added. The following formula (II) represents an ionization reaction of the aggregation-induced phosphor (TPE-COOH) having the carboxyl group. The reaction is an equilibrium reaction.

TPE-COOH ⇔ TPE-COO⁻ + H⁺ (II)

The freshness label 11 is, as the freshness label with the sealing member 10, disposed in the vicinity of food such as fresh food or the like, and used in a state of being sealed together with the food. FIG. 8 illustrates an example of how to use the freshness label with the sealing member 10. FIG. 8 is a perspective view illustrating an example of a packaged food including the freshness label with the sealing member 10 according to the embodiment. A packaged food 40 illustrated in FIG. 8 includes the freshness label with the sealing member 10, food P1, a tray T supporting the freshness label with the sealing member 10 and the food P1, and a wrap film which is not illustrated. The freshness label with the sealing member 10 and the food P1 are sealed by the tray T and the wrap film which is not illustrated not to contact the outside air. That is, the tray T and the wrap film form an airtight container, and the freshness label with the sealing member 10 and the food P1 are sealed in the airtight container. The freshness label with the sealing member 10 may be disposed on the tray T as illustrated in FIG. 8, or may be attached to an inner surface of the wrap film.

A type of the food P1 is not limited, and the freshness label with the sealing member 10 is desirably used as a freshness label with a sealing member for food containing water such as marine product, livestock product, or the like. The freshness label with the sealing member 10 can accurately evaluate the freshness without being affected by the drip from the food P1.

As described hereinafter, the packaged food 40 can quantify the freshness of the food P1 by irradiating the freshness label with the sealing member 10 with excitation light such as ultraviolet rays or the like and measuring an intensity (brightness) of its fluorescence. Here, as an example, the target component generated as the freshness of the food P1 deteriorates is an acidic component, and it is assumed that the aggregation-induced phosphor 3a contains an acidic functional group as a polar functional group.

If the food P1 is in a fresh state, concentration of the target component in the atmosphere inside the airtight container is low. Here, an influence of the target component on the molecular arrangement of the aggregation-induced phosphor 3a is small. Therefore, here, the aggregation-induced phosphor 3a does not emit fluorescence with high intensity even when irradiated with the excitation light.

If the freshness of the food P1 decreases, the concentration of the target component in the atmosphere inside the airtight container increases. If the concentration of the target component in the atmosphere increases, a part thereof dissolves in water contained in the freshness label 11. The aqueous solution has the same polarity as the polar functional group of the aggregation-induced phosphor 3a, such that as the concentration of the target component in the aqueous solution increases, affinity or solubility of the aggregation-induced phosphor 3a with respect to the aqueous solution decreases. Therefore, as the concentration of the target component in the atmosphere increases, the molecular arrangement of the aggregation-induced phosphor 3a approaches a state in which water does not exist. Therefore, it is considered that if the freshness of the food P1 decreases, the intensity of the fluorescence emitted by the aggregation-induced phosphor 3a by irradiating the aggregation-induced phosphor 3a with the excitation light increases.

For example, an ultraviolet (UV) lamp is used to irradiate the freshness label with the sealing member 10 with excitation light. A wavelength of ultraviolet rays varies depending on a type of the aggregation-induced phosphor 3a, and according to one example, the wavelength thereof is 350 nm or more and 530 nm or less. For measurement of the fluorescence intensity, for example, the photodetector or the imaging element is used as described above. For example, first, a fluorescent image is captured by using a digital camera or the like while irradiating the freshness label with the sealing member 10 with the UV lamp. Next, by using image processing software, each gradation value of RBG of the fluorescent image is obtained. From the gradation values, the fluorescence intensity of the freshness label 10 can be quantified. For example, an arithmetic average of the gradation values can be used as the fluorescence intensity.

As described above, if the one including the acidic functional group is used as the aggregation-induced phosphor 3a and the target component is the acidic component, the fluorescence of the freshness label 11 becomes higher as the concentration of the target component increases. On the other hand, if the one including the basic functional group is used as the aggregation-induced phosphor 3a and the target component is the basic component, the fluorescence of the freshness label 11 becomes higher as the concentration of the target component increases.

As described above, if the freshness label with the sealing member 10 is used, the freshness of the food P1 can be quantified by a simple method for measuring the intensity (brightness) of the fluorescence by irradiating the freshness label with the sealing member 10 with the excitation light such as the ultraviolet rays or the like. The freshness can be quantified with high accuracy. Hereinafter, the reason will be described.

As described above, with respect to the food P1 for which the freshness label with the sealing member 10 is desired to be used, for example, fresh food generally has high water content. Therefore, the water vapor pressure in the airtight container rises with the lapse of time and finally reaches a saturated water vapor pressure.

When using a freshness label containing a solution in which the aggregation-induced phosphor is dissolved in an organic solvent, the water content of the freshness label eventually becomes an approximately constant value, but decreases immediately after the freshness label is sealed in the airtight container, and increases with the lapse of time. It is because an increase in water vapor pressure, which is caused by allowing the fresh food sealed in the airtight container together with the freshness label to release water into a gas phase, continues until the saturated water vapor pressure is reached. If the water content of the freshness label increases, as described above, the molecular arrangement of the aggregation-induced phosphor changes, which results in a change in fluorescence intensity of the freshness label. Therefore, in the freshness label using the organic solvent, a magnitude of an influence of water on the fluorescence intensity changes during a period from if the freshness label is sealed in the airtight container until the water vapor pressure reaches the saturated water vapor. Therefore, if such a freshness label is used, the freshness cannot be accurately evaluated.

The freshness label provided in the freshness label with the sealing member according to the embodiment contains water. Food such as fresh food or the like releases water into the gas phase. Therefore, during the period from if the freshness label is sealed in the airtight container until the water vapor pressure reaches the saturated water vapor, a difference between an amount of water that evaporates from the freshness label and an amount of water that is supplied from the gas phase to the freshness label is small. Next, after the water vapor pressure reaches the saturated water vapor, the amount of water that evaporates from the freshness label becomes equal to the amount of water that is supplied from the gas phase to the freshness label. Therefore, the water content of the freshness label is kept constant immediately after the freshness label is sealed in the airtight container. Therefore, according to the freshness label provided in the freshness label with the sealing member according to the embodiment, the change in fluorescence intensity caused by the change in water content hardly occurs.

In the freshness label with the sealing member according to the embodiment, the freshness label is sealed with the sealing member that is impermeable to aqueous liquid from the surrounding atmosphere, thereby preventing aqueous liquid from entering the sealing member. Therefore, for example, the freshness label is not covered with a drip released from fresh food in the airtight container, and water, ice water and seawater which are used to keep the temperature low during the transportation of fresh fish, fish and shellfish, or the like. Therefore, if such freshness label with the sealing member is used, the fluorescence intensity is affected only by a substance which is generated by decomposition or deterioration of a component contained in food and diffuses into a gas phase.

As described above, the freshness label with the sealing member according to the embodiment can implement a simple and highly accurate freshness evaluation. Therefore, if the freshness label with the sealing member according to the embodiment is used, it is possible not only to detect the presence of the target component but also to grasp the concentration and K value of the target component. That is, the fluorescence intensity of the freshness label with the sealing member according to the embodiment can indicate a positive correlation with the concentration of the target component and the K value.

The K value is one of the indexes indicating the freshness of food, and a large K value indicates that the freshness is low. The K value is calculated from an amount of a decomposition product generated by a nucleic acid-related substance over time.

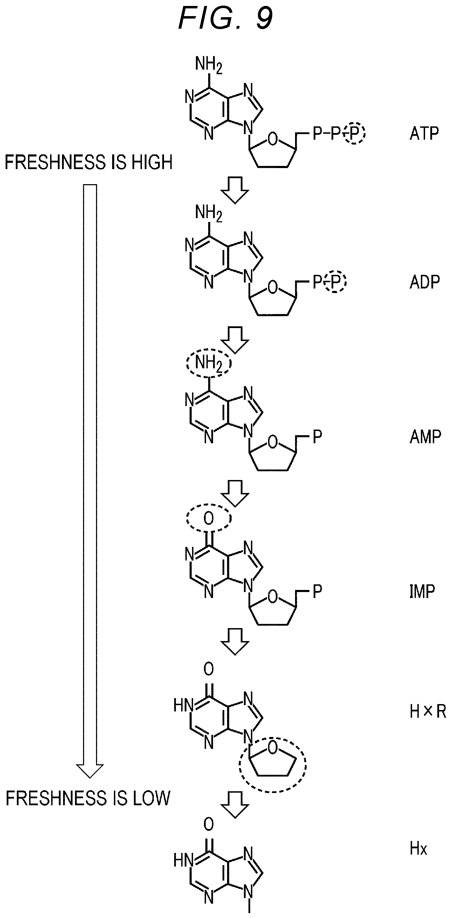

FIG. 9 is an explanatory diagram illustrating a decomposition process of a nucleic acid-related substance. As illustrated in FIG. 9, adenosine triphosphate (ATP) contained in food is decomposed into adenosine diphosphate (ADP). ADP is decomposed into adenosine monophosphate (AMP). AMP is decomposed into inosine monophosphate (IMP). IMP is decomposed to inosine (HxR). HxR is decomposed into hypoxanthine (Hx). ATP, ADP, AMP, and IMP are substances contained in food with high freshness, and HxR and Hx are substances contained in food with low freshness.

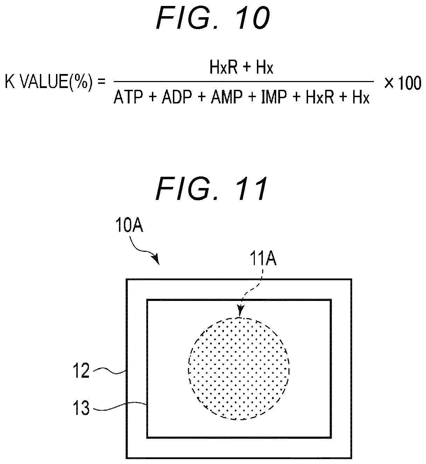

FIG. 10 is a diagram illustrating a method for calculating the K value. As illustrated in FIG. 10, the K value represents a ratio of a total amount of HxR and Hx to a total amount of ATP, ADP, AMP, IMP, HxR, and Hx as a percentage. The amount of ATP, ADP, AMP, IMP, HxR, and Hx in food is calculated, for example, by high-speed liquid chromatography or electrophoresis analysis. Generally, if the K value is 60% or more, the food is determined to be putrefied. Evaluation by K value can indicate the freshness of food relatively accurately. However, in order to calculate the K value, it is required to collect a sample from food and it is also required to use an expensive analytical device.

As described above, the fluorescence intensity of the freshness label with the sealing member according to the embodiment has a positive correlation with the concentration of the target component. The concentration of the target component has a positive correlation with the K value. Therefore, if the fluorescence intensity of the freshness label is quantified and a calibration curve indicating a relationship between the value and the concentration of the target component or the K value is prepared in advance, by measuring the fluorescence intensity and referring to a measurement result thereof in the calibration curve, the concentration of the target component in the gas phase or the K value of the food can be obtained.

Next, another usage method of the freshness label according to the embodiment will be described. FIG. 11 is a top view schematically illustrating another example of the freshness label with the sealing member according to the embodiment. A freshness label with a sealing member 10A illustrated in FIG. 11 has the same structure as that of the freshness label with the sealing member 10 illustrated in FIG. 3, except that a freshness label 11A further contains acid which is not illustrated. That is, the freshness label with the sealing member 10A illustrated in FIG. 11 includes the freshness label 11A and a sealing member formed of a retaining body 12, a porous member 13, and a joining member which is not illustrated in a form of sealing the freshness label 11A from the surrounding atmosphere. The freshness label 11A contains the freshness label structure 1 illustrated in FIGS. 1 and 2, and the water carried on the freshness label structure 1 and the acid. In other words, the freshness label 11A carries an acidic aqueous solution. As the acid, for example, formic acid, hydrochloric acid, acetic acid, or a mixture thereof are used. From a viewpoint of safety, it is desirable to use acetic acid as the acid. The polar functional group of the aggregation-induced phosphor 3a is desirably the acidic functional group. Here, as an example, it is assumed that the polar functional group of the aggregation-induced phosphor 3a is the acidic functional group.

The freshness label with the sealing member 10A illustrated in FIG. 11 can be obtained, for example, by immersing the freshness label with the sealing member 10 illustrated in FIG. 3 in an aqueous solution containing a high concentration of acid for a predetermined time. The acid content in the freshness label with the sealing member 10A can be adjusted depending on a desired fluorescence intensity.

In the freshness label with the sealing member 10A illustrated in FIG. 11, since the freshness label 11A contains acid, it is considered that the molecular arrangement of the aggregation-induced phosphor 3a is close to a water-free state, or that the conformation of the aggregation-induced phosphor 3a is changed due to the presence of an acid component. Therefore, the freshness label with the sealing member 10A shows fluorescence stronger than that of the freshness label with the sealing member 10 illustrated in FIG. 3.

The fluorescence intensity of the freshness label with the sealing member 10A decreases if the freshness label with the sealing member 10A contacts a basic target component, and becomes non-fluorescent when contacting a certain amount or more of the basic target component. That is, the fluorescence intensity of the freshness label with the sealing member 10A illustrated in FIG. 11 can show a negative correlation with the concentration of the target component and the K value. Therefore, in the same manner as that of the freshness label with the sealing member 10 illustrated in FIG. 3, if the fluorescence intensity of the freshness label with the sealing member 10A is quantified and the calibration curve indicating the relationship between the value and the concentration of the target component or the K value is prepared in advance, by measuring the fluorescence intensity and referring to the measurement result thereof in the calibration curve, the concentration of the target component in the gas phase or the K value of the food can be obtained.

The freshness label with the sealing member may be distributed in a state of not containing water or the like, and after that, for example, the freshness label with the sealing member may be impregnated with water or the like at a location where the freshness label with the sealing member is sealed in the airtight container together with the food P1. Here, the freshness label structure with the sealing member 100 may be distributed as a freshness label kit including the freshness label structure with the sealing member 100 and one or more liquids to be contained therein, that is, water, acid, or a combination of water and acid. Alternatively, the freshness label structure with the sealing member 100 may be divided into the sealing member and the freshness label structure, and may be further distributed as a freshness label kit containing one or more liquids to be contained in the freshness label structure. If the combination of water and acid is contained in the freshness label kit, water and acid may be contained in separate containers, or may be mixed and contained as an aqueous solution in a single container.

Alternatively, the freshness label with the sealing member 10 or 10A, in which the freshness label structure with the sealing member 100 is impregnated with water or the like, may be distributed and sealed in the airtight container together with the food P1.

The freshness label structure, provided in the freshness label structure with the sealing member 100 according to the embodiment, includes the phosphor layer fixed to the base material. Therefore, the freshness label can be manufactured by impregnating the freshness label structure with water. Since the freshness label containing water is less likely to be affected by water in the gas phase, the freshness of food can be evaluated with higher accuracy than the freshness label containing the organic solvent. The freshness label with the sealing member according to the embodiment has the waterproof structure, and aqueous liquid existing in the environment surrounding the freshness label does not affect the fluorescence intensity, such that it is possible to evaluate the freshness of food with high accuracy in various environments where aqueous liquid exists.

### [Embodiments]

### [First embodiment]

In the embodiment, the freshness label with the sealing member 10 and the freshness label with the sealing member 10A are manufactured, and the freshness label with the sealing member 10A is used to evaluate the freshness of fresh fish as an initial freshness label state (an initial state) for evaluating the freshness of food.

The freshness label with the sealing member 10 illustrated in FIGS. 3 and 4 is manufactured by the following method. First, the aggregation-induced phosphor 3a is dissolved in ethanol to manufacture a treatment liquid having concentration of 200 µM. After immersing the base material 2 in the treatment liquid, the base material 2 is pulled up from the treatment liquid, placed on the retaining body 12, and dried. As the aggregation-induced phosphor 3a, the compound represented by the structural formula (6) is used. As the base material 2, commercially available glass filter paper (GS-25; Advantech Co., Ltd.) is used. As the retaining body 12, a glass plate is used. As such, the freshness label structure 1 illustrated in FIG. 1 is obtained.

Next, the joining member 14 is disposed to surround the dried freshness label structure 1, and the hydrophobic porous member 13 is stacked thereon. A thermocompression bonding film (Fixeron (registered trademark); Aicello Corporation) is used as the joining member 14, and a PTFE filter (a Mitex (registered trademark) membrane filter; a pore size of 5.0 µm; Merck Millipore Corporation) is used as the hydrophobic porous member 13. The obtained stacked body is placed on a hot plate heated to 200°C, weight is placed on the hot plate, and pressure is applied thereto for 2 minutes, such that the porous member 13 and the retaining body 12 closely contact each other to obtain a freshness label structure with a sealing member 100. If the freshness label structure with the sealing member 100 is observed while being irradiated with ultraviolet ray by a UV lamp, it is confirmed that the freshness label structure with the sealing member 100 emits strong fluorescence. A wavelength of the ultraviolet ray emitted by the UV lamp is 365 nm.

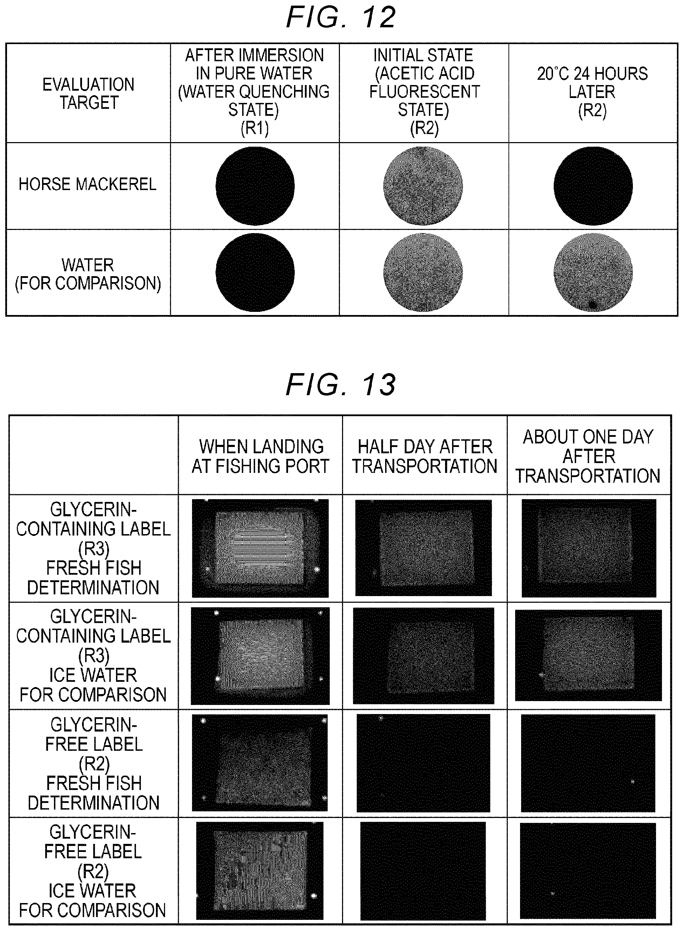

Next, the freshness label structure with the sealing member 100 is immersed in pure water. After being immersed therewith for half a day, the freshness label structure with the sealing member 100 is pulled out from the pure water. Here, the freshness label structure 1 becomes the freshness label 11 by absorbing water through the hydrophobic porous member 13, thereby obtaining the freshness label with the sealing member 10 illustrated on FIGS. 3 and 4. Hereinafter, the freshness label with the sealing member 10 will be referred to as a freshness label with a sealing member R1. If the freshness label with the sealing member R1 is observed while being irradiated with the ultraviolet ray by the UV lamp, it is confirmed that freshness label with the sealing member R1 is in a non-fluorescent quenching state. The freshness label with the sealing member R1 here is photographed by using a digital camera, and a photographed image thereof is recorded by using digital image data. The image is illustrated in FIG. 12.

The obtained freshness label with the sealing member R1 is immersed in acetic acid water having concentration of several vol% for about one hour, and then pulled out therefrom. Here, the freshness label 11 absorbs water and acetic acid through the hydrophobic porous member 13, thereby obtaining the freshness label with the sealing member 10A illustrated in FIG. 11. Hereinafter, the freshness label with the sealing member 10A will be referred to as a freshness label with a sealing member R2. If the freshness label with the sealing member R2 is observed while being irradiated with the ultraviolet ray by the UV lamp, it is confirmed that the freshness label with the sealing member R2 emits strong fluorescence. The freshness label with the sealing member R2 here is photographed by using a digital camera, a photographed image thereof is recorded by using digital image data. The image is illustrated in FIG. 12.

The freshness label with the sealing member R2 obtained as described above is used to evaluate the freshness of fresh fish by the following method as an initial freshness label state (an initial state) for evaluating the freshness of food.

The two freshness labels with sealing members R2 are respectively placed and sealed in a plastic container containing fish meat pieces of a horse mackerel and a plastic container containing pure water for comparison. The containers are placed in a constant temperature bath set at 20°C. After 20 hours, the freshness label with the sealing member R2 is taken out from each container and observed while being irradiated with ultraviolet ray with the UV lamp to confirm the fluorescence intensity. The freshness label with the sealing member R2 here is photographed with a digital camera, and a photographed image thereof is recorded by using digital image data. The image is illustrated in FIG. 12.

As illustrated in the image of FIG. 12, the fluorescence intensity of the freshness label with the sealing member R2 placed in the container containing a horse mackerel is significantly lower than the fluorescence intensity in the initial state before the test. Therefore, it can be seen that the fluorescence intensity is lowered by reaction between the target component generated as the freshness of a horse mackerel deteriorates and the freshness label. On the other hand, the fluorescence intensity of the freshness label with the sealing member R2 placed in the container containing pure water is approximately the same as the fluorescence intensity in the initial state. As described above, it can be seen that lowering of the fluorescence cannot be confirmed in pure water, such that freshness determination ability of the freshness label with the sealing member R2 is maintained.

### [Second embodiment]

In the embodiment, the waterproof function of the freshness label with the sealing member is evaluated.

As the freshness label with the sealing member, the freshness label with the sealing member R2 manufactured in the first embodiment is used. The freshness label with the sealing member R2 is placed in a container containing pure water to the extent that the freshness label with the sealing member R2 is submerged and immersed in pure water. After one hour in such state, the freshness label with the sealing member R2 is taken out from the pure water and observed while being irradiated with the ultraviolet ray by the UV lamp to confirm the fluorescence intensity. The freshness label with the sealing member R2 here is photographed with a digital camera, and a photographed image thereof is recorded by using digital image data. As a result, the fluorescence intensity of the freshness label with the sealing member R2 is approximately the same as the fluorescence intensity in the initial state of the freshness label with the sealing member R2 before immersion. As described above, it is confirmed that the freshness label with the sealing member R2 can maintain the waterproof function against aqueous liquid and a fluorescence mechanism as the freshness label.

As a result of observing the freshness label with the sealing member R2 after the freshness label with the sealing member R2 is immersed in pure water in the above-described state for one week, deterioration such as peeling or the like is not confirmed in the hydrophobic porous member 13. It is confirmed that the waterproof property for repelling water droplets also does not deteriorate.

### [Third embodiment]

In the embodiment, an effect of a freshness label with a sealing member containing a moisturizer is evaluated. The freshness label with the sealing member containing the moisturizer is appropriately used for freshness management of fresh fish, for example, if landed fresh fish is packed in a box and delivered from a fishing port to a market and a customer through a transportation process by a truck or the like. Normally, ice, water, seawater, or the like are put in the box as a cold insulating material together with fresh fish, and water droplets and ice blocks are scattered due to vibration during the transportation. Therefore, in order to manage the freshness of fresh fish by using the freshness label in the cold storage box, it is required to provide a waterproof structure to the freshness label so that even though a certain amount of water and ice blocks cover the freshness label, a fluorescence emission state is not affected. It is also required that an initial fluorescence emission state can be maintained while the freshness of the fresh fish is maintained well after the transportation of the fresh fish.

Therefore, in the embodiment, the fluorescent state of the freshness label with the sealing member containing the moisturizer and the freshness label with the sealing member not containing the moisturizer is evaluated in the actual situation by using the cold storage box including fresh fish and ice water.

In the embodiment, the freshness label with the sealing member R2 manufactured in the first embodiment is used as the freshness label with the sealing member that does not contain the moisturizer. The freshness label with the sealing member containing the moisturizer uses glycerin as the moisturizer and is manufactured as follows. First, in the method for manufacturing the freshness label with the sealing member R2 of the first embodiment, glycerin is added to the treatment liquid containing the aggregation-induced phosphor 3a and ethanol prepared for manufacturing the freshness label structure 1. Glycerin is added so that the concentration in the treatment liquid becomes 5% by mass. Otherwise, the freshness label with the sealing member containing the moisturizer is manufactured by the same method as the manufacturing method of the freshness label with the sealing member R2. The obtained freshness label with the sealing member containing the moisturizer is referred to as a freshness label with a sealing member R3.

The two freshness labels with sealing members R3 are respectively installed and sealed in a cold storage box including fresh fish immediately landed from a fishing port and ice water, and in a cold storage box including only ice water for comparison. The two freshness labels with sealing members R2 are respectively installed and sealed in a cold storage box including fresh fish immediately landed from a fishing port and ice water, and in a cold storage box including only ice water for comparison. The four types of cold storage boxes are transported by truck from the fishing port to a market for about two hours, moved to a wholesaler in the market, and further transported by truck to a prospective customer in about two hours. Each cold storage box after transportation is kept in a refrigerated state without melting the ice water until about one day elapses after landing and transportation.

Before transportation (at the time of landing at the fishing port), after half a day after the transportation, and after about one day after the transportation, the freshness label with the sealing member is taken out from each cold storage box and observed while being irradiated with the ultraviolet ray by the UV lamp to confirm the fluorescence intensity. The respective freshness labels with the sealing members here are photographed by using a digital camera and a photographed image is recorded by using digital image data. The image is illustrated in FIG. 13. From FIG. 13, it can be seen that the freshness label with the sealing member R3 containing glycerin has almost the same fluorescence intensity as that before the transportation even after about one day elapses after the transportation in both fresh fish determination and comparison, and is in a state where the freshness can be determined. On the other hand, the freshness label with the sealing member R2 that does not contain glycerin shows a phenomenon of fluorescence quenching in both the fresh fish determination and the comparison. Since the phenomenon appears in the only ice water for comparison, it is presumed that concentration of acid components starts to decrease from the freshness label in the freshness label with the sealing member R2. That is, if a vibration state such as cold storage box transportation continues for a long time without a moisturizing component, it is presumed that water flow in and out of the freshness label also frequently occurs, and at the same time, water content concentration in the freshness label rises with an inflow and outflow of the acid component, such that the quenching effect occurs without reaching freshness determination. It is considered to be a phenomenon peculiar to a case such as the cold storage box transportation. A freshness state of the fresh fish after about one day indicates 10% or less in the K value determination, and a good freshness state is maintained.

## Claims

1. A freshness label structure (10, 20, 30) comprising:
a label structure (1) including :
a base material (2) which is a fibrous material; and
a phosphor layer (3) carried on a fiber (2a) of the base material (2a), the phosphor layer including an aggregation-induced phosphor (3a) fixed to the fiber (2a) of the base material; and
either water or water and acid, wherein the label structure (1) does not contain an organic solvent, and
a sealing member (100) that is impermeable to aqueous liquid, seals the label structure, and includes a porous member (13) impermeable to aqueous liquid and permeable to gas.

2. The freshness label structure of claim 1, wherein the label structure includes water.

3. The freshness label structure of claim 1 or 2, wherein the label structure includes water and acid.

4. The freshness label structure of claim 3, wherein the phosphor layer is in a fluorescent state through interaction with the water and the acid.

5. The freshness label structure of claim 4, wherein an interaction of the aggregation-induced phosphor with the water and the acid is disrupted by exposure of the water, the acid, or the aggregation-induced phosphor to a target component from degrading food, thereby removing the aggregation-induced phosphor from the fluorescent state.

6. The freshness label structure according to any one of claims 1 to 5, wherein the label structure includes a moisturizer.

7. The freshness label structure according to any one of claims 1 to 6, wherein:
the base material has a thickness of between 0.1 mm and 1.0 mm; and
the phosphor layer has a thickness of less than 30 nm.

8. The freshness label structure according to any one of claims 1 to 7, wherein:
the porous member is a hydrophobic polytetrafluoroethylene membrane filter.

9. The freshness label structure according to any one of claims 1 to 8, wherein the sealing member includes a retaining body coupled to the porous member using an adhesive, and wherein the label structure is disposed between the retaining body and the porous member.

10. The freshness label structure according to any one of claims 1 to 8, wherein the sealing member further includes a retaining body and a film, the porous member being positioned between the retaining body and the film, and an adhesive coupling (i) the porous member to the retaining body, (ii) the porous member to the film, and (iii) the retaining body to the film, and wherein the label structure is disposed between the retaining body and the film.

11. The freshness label structure according to any one of claims 1 to 8, wherein the sealing member further includes a retaining body, a film, and an adhesive coupling the retaining body to the film,
wherein one of the film or the retaining body define an opening,
wherein the porous member is disposed in the opening, and
wherein the label structure is disposed between the retaining body and the film.

12. The freshness label structure according to any one of claims 1 to 11, wherein the sealing member includes:
a first housing defining a peripheral groove and an internal space that receives the label structure;
a second housing having a bottom wall and a peripheral wall extending from the bottom wall, the bottom wall defining an opening, the peripheral wall received within the peripheral groove of the first housing; and
the porous member positioned between the first housing and the second housing.

13. The freshness label structure according to any one of claims 1 to 12, wherein the aggregation-induced phosphor comprises one or more polar functional groups.

14. The freshness label structure according to any one of claims 1 to 13, wherein the aggregation-induced phosphor comprises at least one compound selected from a tetra-phenylethylene represented by the following formula (I): 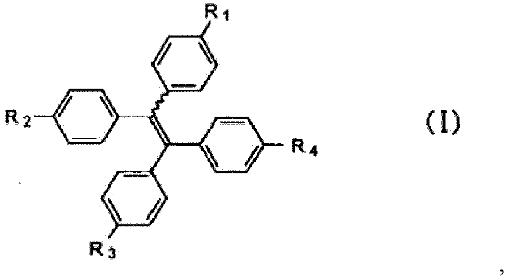
wherein R₁, R₂, R₃, and R₄ are independently selected from a group selected from: a hydrogen atom, a halogen atom, a hydroxyl group, a nitro group, a carbamoyl group, an alkyl groups having a carbon number of 1 to 6, a haloalkyl group having a carbon number of 1 to 6, an alkenyl group having a carbon number of 2 to 6, a cycloalkyl group having a carbon number of 3 to 10, an alkyloxy group having a carbon number of 1 to 6, an acyl group having a carbon number of 2 to 6, an amino group, an alkylamino group having a carbon number of 1 to 6, an aryl group having a carbon number of 6 to 10, a heteroaryl group having a carbon number of 5 to 10, L₁M₁, -(CH₂)ₘ-L₂M₂, -X-(CH₂)ₙ-L₃M₃, and -Y-(CH₂)ₒ-Z-(CH₂)ₚ-L₄M₄,
wherein:
L₁, L₂, L₃, and L₄ are independent of each other and represent -CO₂- or - SO₃-;
M₁, M₂, M₃, and M₄ are independent of each other and represent a hydrogen atom or a cation;
X, Y, and Z are independent of each other and represent -O-, -NH-, or -S-;
m, n, o, and p are independent of each other and represent integers 1 to 6.

15. The freshness label structure according to any one of claims 1 to 14, wherein the aggregation-induced phosphor comprises at least one compound selected from formulas (5), (6), (7), and (8) below:

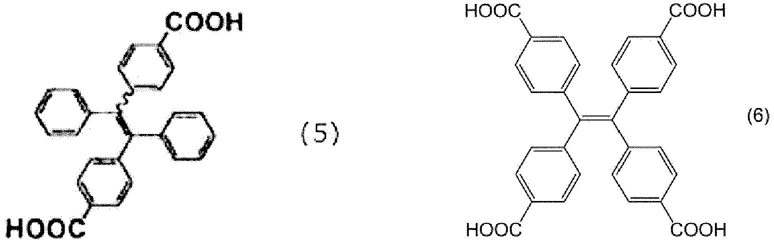

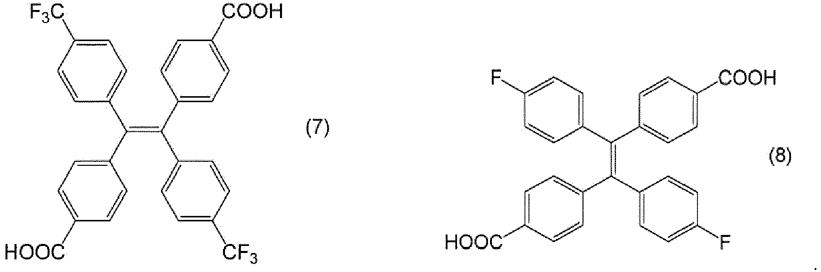

## Patentansprüche

1. Frischeetikettstruktur (10, 20, 30), umfassend:
eine Etikettstruktur (1), die Folgendes beinhaltet:
ein Grundmaterial (2), das ein faseriges Material ist; und
eine Leuchtstoffschicht (3), die auf einer Faser (2a) des Grundmaterials (2a) getragen wird, wobei die Leuchtstoffschicht einen aggregationsinduzierten Leuchtstoff (3a) beinhaltet, der an der Faser (2a) des Grundmaterials befestigt ist; und
entweder Wasser oder Wasser und Säure, wobei die Etikettstruktur (1) kein organisches Lösungsmittel enthält, und
ein Dichtungselement (100), das für wässrige Flüssigkeit undurchlässig ist, die Etikettstruktur abdichtet und ein poröses Element (13) beinhaltet, das für wässrige Flüssigkeit undurchlässig und für Gas durchlässig ist.

2. Frischeetikettstruktur nach Anspruch 1, wobei die Etikettstruktur Wasser beinhaltet.

3. Frischeetikettstruktur nach Anspruch 1 oder 2, wobei die Etikettstruktur Wasser und Säure beinhaltet.

4. Frischeetikettstruktur nach Anspruch 3, wobei die Leuchtstoffschicht durch Wechselwirkung mit dem Wasser und der Säure in einem fluoreszierenden Zustand ist.

5. Frischeetikettstruktur nach Anspruch 4, wobei eine Wechselwirkung des aggregationsinduzierten Leuchtstoffs mit dem Wasser und der Säure gestört wird, indem das Wasser, die Säure oder der aggregationsinduzierte Leuchtstoff einer Zielkomponente aus einem sich zersetzenden Lebensmittel ausgesetzt wird, wodurch der aggregationsinduzierte Leuchtstoff aus dem fluoreszierenden Zustand entfernt wird.

6. Frischeetikettstruktur nach einem der Ansprüche 1 bis 5, wobei die Etikettstruktur ein Befeuchtungsmittel beinhaltet.

7. Frischeetikettstruktur nach einem der Ansprüche 1 bis 6, wobei:
das Grundmaterial eine Stärke zwischen 0,1 mm und 1,0 mm aufweist; und
die Leuchtstoffschicht eine Stärke von weniger als 30 nm aufweist.

8. Frischeetikettstruktur nach einem der Ansprüche 1 bis 7, wobei:
das poröse Element ein hydrophober Polytetrafluorethylen-Membranfilter ist.

9. Frischeetikettstruktur nach einem der Ansprüche 1 bis 8, wobei das Dichtungselement einen Haltekörper beinhaltet, der unter Verwendung eines Klebers mit dem porösen Element gekoppelt ist, und wobei die Etikettstruktur zwischen dem Haltekörper und dem porösen Element angeordnet ist.

10. Frischeetikettstruktur nach einem der Ansprüche 1 bis 8, wobei das Dichtungselement ferner einen Haltekörper und einen Film umfasst, wobei das poröse Element zwischen dem Haltekörper und dem Film angeordnet ist, und ein Kleber (i) das poröse Element mit dem Haltekörper, (ii) das poröse Element mit dem Film und (iii) den Haltekörper mit dem Film koppelt, und wobei die Etikettstruktur zwischen dem Haltekörper und dem Film angeordnet ist.

11. Frischeetikettstruktur nach einem der Ansprüche 1 bis 8, wobei das Dichtungselement ferner einen Haltekörper, einen Film und einen Kleber, der den Haltekörper mit dem Film verbindet, beinhaltet,
wobei eines von dem Film oder dem Haltekörper eine Öffnung definiert,
wobei das poröse Element in der Öffnung angeordnet ist, und
wobei die Etikettstruktur zwischen dem Haltekörper und dem Film angeordnet ist.

12. Frischeetikettstruktur nach einem der Ansprüche 1 bis 11, wobei das Dichtungselement Folgendes beinhaltet:
ein erstes Gehäuse, das eine Umfangsrille und einen Innenraum, der die Etikettstruktur aufnimmt, definiert;
ein zweites Gehäuse, das eine Bodenwand und eine Umfangswand, die sich von der Bodenwand erstreckt, aufweist, wobei die Bodenwand eine Öffnung definiert, die Umfangswand in der Umfangsrille des ersten Gehäuses aufgenommen ist; und
wobei das poröse Element zwischen dem ersten Gehäuse und dem zweiten Gehäuse positioniert ist.

13. Frischeetikettstruktur nach einem der Ansprüche 1 bis 12, wobei der aggregationsinduzierte Leuchtstoff eine oder mehrere polare funktionelle Gruppen umfasst.

14. Frischeetikettstruktur nach einem der Ansprüche 1 bis 13, wobei der aggregationsinduzierte Leuchtstoff mindestens eine Verbindung umfasst, die ausgewählt ist aus einem Tetraphenylethylen, das durch die folgende Formel (I) dargestellt ist: 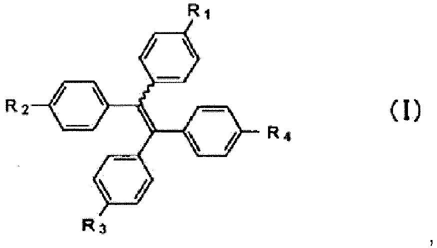
wobei R₁, R₂, R₃ und R₄ unabhängig ausgewählt sind aus einer Gruppe, die ausgewählt ist aus: einem Wasserstoffatom, einem Halogenatom, einer Hydroxylgruppe, einer Nitrogruppe, einer Carbamoylgruppe, einer Alkylgruppe, die eine Kohlenstoffzahl von 1 bis 6 aufweist, einer Haloalkylgruppe, die eine Kohlenstoffzahl von 1 bis 6 aufweist, einer Alkenylgruppe, die eine Kohlenstoffzahl von 2 bis 6 aufweist, einer Cycloalkylgruppe, die eine Kohlenstoffzahl von 3 bis 10 aufweist, einer Alkyloxygruppe, die eine Kohlenstoffzahl von 1 bis 6 aufweist, einer Acylgruppe, die eine Kohlenstoffzahl von 2 bis 6 aufweist, einer Aminogruppe, einer Alkylaminogruppe, die eine Kohlenstoffzahl von 1 bis 6 aufweist, einer Arylgruppe, die eine Kohlenstoffzahl von 6 bis 10 aufweist, einer Heteroarylgruppe, die eine Kohlenstoffzahl von 5 bis 10 aufweist, L₁M₁, -(CH₂)ₘ-L₂M₂, -X-(CH₂)ₙ-L₃M₃ und -Y-(CH₂)₀-Z-(CH₂)ₚ-L₄M₄,
wobei:
L₁, L₂, L₃ und L₄ voneinander unabhängig sind und -CO₂- oder --SOsdarstellen;
M₁, M₂, M₃ und M₄ voneinander unabhängig sind und ein Wasserstoffatom oder ein Kation darstellen;
X, Y und Z voneinander unabhängig sind und -O-, -NH- oder -S- darstellen;
m, n, o und p voneinander unabhängig sind und ganze Zahlen 1 bis 6 darstellen.

15. Frischeetikettstruktur nach einem der Ansprüche 1 bis 14, wobei der aggregationsinduzierte Leuchtstoff mindestens eine Verbindung umfasst, die ausgewählt ist aus Formel (5), (6), (7) und (8) unten:

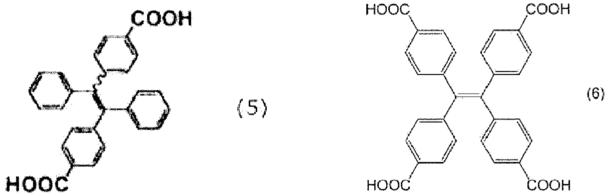

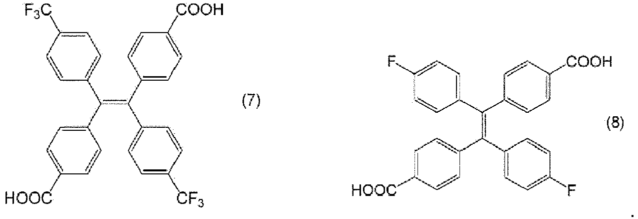

## Revendications

1. Structure d'étiquette fraîcheur (10, 20, 30) comprenant :
une structure d'étiquette (1) contenant :
un matériau de base (2) qui est un matériau fibreux ; et
une couche de luminophore (3) portée sur une fibre (2a) du matériau de base (2a), la couche de luminophore contenant un luminophore induit par agrégation (3a) fixé à la fibre (2a) du matériau de base ; et
soit de l'eau soit de l'eau et un acide, dans laquelle la structure d'étiquette (1) ne contient pas de solvant organique, et
un élément d'étanchéité (100) qui est imperméable aux liquides aqueux, scelle la structure d'étiquette, et contient un élément poreux (13) imperméable aux liquides aqueux et perméable aux gaz.

2. Structure d'étiquette fraîcheur selon la revendication 1, dans laquelle la structure d'étiquette contient de l'eau.

3. Structure d'étiquette fraîcheur selon la revendication 1 ou 2, dans laquelle la structure d'étiquette contient de l'eau et un acide.

4. Structure d'étiquette fraîcheur selon la revendication 3, dans laquelle la couche de luminophore est dans un état fluorescent par l'interaction avec l'eau et l'acide.

5. Structure d'étiquette fraîcheur selon la revendication 4, dans laquelle une interaction du luminophore induit par une agrégation avec l'eau et l'acide est interrompue par l'exposition de l'eau, de l'acide ou du luminophore induit par agrégation à un composant cible issu d'un aliment en cours de dégradation, en retirant ainsi le luminophore induit par agrégation de son état fluorescent.

6. Structure d'étiquette fraîcheur selon l'une quelconque des revendications 1 à 5, dans laquelle la structure d'étiquette contient un hydratant.

7. Structure d'étiquette fraîcheur selon l'une quelconque des revendications 1 à 6, dans laquelle :
le matériau de base a une épaisseur comprise entre 0,1 mm et 1,0 mm ; et
la couche de luminophore a une épaisseur inférieure à 30 nm.

8. Structure d'étiquette fraîcheur selon l'une quelconque des revendications 1 à 7, dans laquelle :
l'élément poreux est un filtre à membrane en polytétrafluoroéthylène hydrophobe.

9. Structure d'étiquette fraîcheur selon l'une quelconque des revendications 1 à 8, dans laquelle l'élément d'étanchéité contient un corps de retenue couplé à l'élément poreux au moyen d'un adhésif, et dans laquelle la structure d'étiquette est disposée entre le corps de retenue et l'élément poreux.

10. Structure d'étiquette fraîcheur selon l'une quelconque des revendications 1 à 8, dans laquelle l'élément d'étanchéité contient en outre un corps de retenue et un film, l'élément poreux étant positionné entre le corps de retenue et le film, et un adhésif couplant (i) l'élément poreux au corps de retenue, (ii) l'élément poreux au film, et (iii) le corps de retenue au film, et dans laquelle la structure d'étiquette est disposée entre le corps de retenue et le film.

11. Structure d'étiquette fraîcheur selon l'une quelconque des revendications 1 à 8, dans laquelle l'élément d'étanchéité contient en outre un corps de retenue, un film, et un adhésif couplant le corps de retenue au film,
dans laquelle l'un parmi le film et le corps de retenue définit une ouverture,
dans laquelle l'élément poreux est disposé dans l'ouverture, et
dans laquelle la structure d'étiquette est disposée entre le corps de retenue et le film.

12. Structure d'étiquette fraîcheur selon l'une quelconque des revendications 1 à 11, dans laquelle l'élément d'étanchéité contient :
un premier logement définissant une rainure périphérique et un espace interne qui reçoit la structure d'étiquette ;
un deuxième logement ayant une paroi inférieure et une paroi périphérique s'étendant depuis la paroi inférieure, la paroi inférieure définissant une ouverture, la paroi périphérique étant reçue à l'intérieur de la rainure périphérique du premier logement ; et
l'élément poreux positionné entre le premier logement et le deuxième logement.

13. Structure d'étiquette fraîcheur selon l'une quelconque des revendications 1 à 12, dans laquelle le luminophore induit par agrégation comprend un ou plusieurs groupes fonctionnels polaires.

14. Structure d'étiquette fraîcheur selon l'une quelconque des revendications 1 à 13, dans laquelle le luminophore induit par agrégation comprend au moins un composé choisi parmi un tétraphényléthylène représenté par la formule (I) suivante : 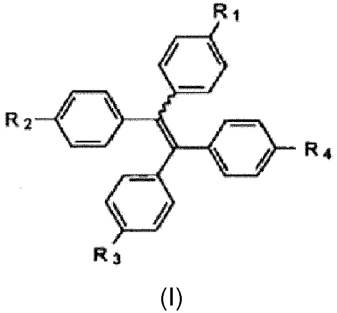
dans laquelle R₁, R₂, R₃ et R₄ sont indépendamment choisis dans l'ensemble constitué par : un atome d'hydrogène, un atome d'halogène, un groupe hydroxyle, un groupe nitro, un groupe carbamoyle, un groupe alkyle ayant 1 à 6 carbones, un groupe halogénoalkyle ayant 1 à 6 carbones, un groupe alcényle ayant 2 à 6 carbones, un groupe cycloalkyle ayant 3 à 10 carbones, un groupe alkyloxy ayant 1 à 6 carbones, un groupe acyle ayant 2 à 6 carbones, un groupe amino, un groupe alkylamino ayant 1 à 6 carbones, un groupe aryle ayant 6 à 10 carbones, un groupe hétéroaryle ayant 5 à 10 carbones, L₁M₁, -(CH₂)ₘ-L₂M₂, -X-(CH₂)ₙ-L₃M₃ et -Y-(CH₂)ₒ-Z-(CH₂)ₚ-L₄M₄,
dans laquelle :
L₁, L₂, L₃ et L₄ sont indépendants les uns des autres et représentent -CO₂- ou -SOs- ;
M₁, M₂, M₃ et M₄ sont indépendants les uns des autres et représentent un atome d'hydrogène ou un cation ;
X, Y et Z sont indépendants les uns des autres et représentent -O-, -NH-, ou -S- ;
m, n, o et p sont indépendants les uns des autres et représentent des entiers de 1 à 6.

15. Structure d'étiquette fraîcheur selon l'une quelconque des revendications 1 à 14, dans laquelle le luminophore induit par agrégation comprend au moins un composé choisi parmi les formules (5), (6), (7) et (8) ci-dessous :
